# EUROPEAN PATENT SPECIFICATION

(11) **EP 0 668 929 B1**
(45) Date of publication and mention of the grant of the patent: **13.08.1997**
(21) Application number: 92911283.7
(22) Date of filing: 05.06.1992
(51) Int. Cl.: C12Q 1/68, C12Q 1/70

(54) **QUANTITATIVE DETECTION OF NUCLEIC ACID AMPLIFICATION PRODUCTS**
QUANTITATIVER NACHWEIS VON NUKLEINSÄURE-AMPLIFIKATIONSPRODUKTEN
DETECTION QUANTITATIVE DES PRODUITS D'AMPLIFICATION D'ACIDE NUCLEIQUE

(30) Priority: 07.06.1991 GB 91122515
(43) Date of publication of application: 30.08.1995
(73) Proprietor: AMERSHAM INTERNATIONAL plc, Little Chalfont Buckinghamshire HP7 9NA (GB)
(72) Inventor: BLAIR, James, Ninian, Rowell 39 Old Church Road, Cardiff CF4 1AA (GB); DOWNES, Malcolm, John, Cardiff CF5 4SG (GB); PARRY, David, Alun Flat 2, Cardiff CF1 3EF (GB); CROSS, Lisa, Heath Cardiff CF4 3PQ (GB); ROBINSON, Philip, Steven 98 Winslow Road Wingrave, Buckinghamshire HP22 4QB (GB)
(74) Representative: Pennant, Pyers
(86) International application number: GB9201014
(87) International publication number: WO9221780

(56) References cited:
- WO-A-89/09835
- WO-A-91/02817
- WO-A-91/08489
- US-A- 4 568 649
- NUCLEIC ACIDS RESEARCH, SYMP. SER. no. 20, 1988, ARLINGTON, VIRGINIA, US; pp. 91 - 92; A. YAMANE ET AL.: 'Rapid detection of specific gene sequences'
- NATURE. vol. 341, 14 September 1989, LONDON GB pages 167 - 168; N. BOSWORTH ET AL.: 'Scintillation proximity assay' cited in the application
- NUCLEIC ACIDS RESEARCH. vol. 17, no. 22, 1989, ARLINGTON, VIRGINIA US pages 9437 - 9446; M. BECKER-ANDRE ET AL.: 'Absolute mRNA quantification using the polymerase chain reaction (PCR). A novel approach by a PCR aided transcript titration assay (PATTY)'
- ANALYTICAL BIOCHEMISTRY vol. 195, May 1991, NEW YORK US pages 105 - 110; P. BALAGUER ET AL.: 'Quantification of DNA sequences obtained by Polymerase Chain Reaction using a bioluminiscent adsorbent'

## Description

The present invention relates to a method for the rapid quantitation of products of nucleic acid amplification processes, particularly the Polymerase Chain Reaction (PCR), and which is readily amenable to automation.

The Polymerase Chain Reaction is a method which can be used to rapidly amplify a specific DNA sequence (1). In PCR the sequence to be amplified ("the target", typically 100-5000 bp) is flanked by a pair of primers typically 20-30bp long, that are complementary to regions of the target but non-complementary to each other. Heat denaturation of the target followed by cooling allows annealing of the primers and subsequent extension by a DNA polymerase. Repeated cycles of denaturation/annealing/extension results in an exponential increase in the product DNA copies from the target. Products of the PCR are usually detected by visualising fluorescence of the amplified DNA on an ethidium bromide stained agarose gel, which gives a qualitative answer to the presence or absence of the target sequence.

With an increasing usage of PCR in assays where quantitative detection is required (2,3) a number of techniques have been developed to quantitate initial target sequence copies. Techniques developed include excision of radiolabelled product bands from a gel (4,5) southern blotting followed by [³²P] probe hybridisation and autoradiography (6,7) dot blotting and [³²P] probe hybridisation (8). Rimstad et. al. (9) used a primer pair in which one primer was biotin labelled, the other ³²P labelled. Following PCR separation of product is achieved by contacting the reaction mix with streptavidin coated magnetic beads which are then washed and resuspended for scintillation counting. Lundberg et. al. (10) similarly used a biotin labelled primer and streptavidin coated magnetic beads to separate PCR products, linked to a competitive colourimetric assay. All of these techniques are laborious and time consuming, with time to quantitate being from 3 to 24 hours depending on the technique. They are all manually intensive and not easily automated.

The method of the present invention overcomes these problems, it is rapid and accurate producing quantitative data typically within 30 minutes and also allowing a rapid throughput of multiple samples. The method does not require any separation or washing stages and is readily automatable, thus lending itself to use in large scale screening programmes of, for example, disease status.

Accordingly, the present invention provides a method of amplifying and quantitating a specific nucleic acid sequence in a sample, by the use of
a) at least one oligonucleotide primer for each strand of the sequence to be amplified, the primers being complementary to part of the sequence strand but not to one another
b) a supply of nucleotides
c) optionally a probe complementary to part of the sequence to be amplified but not to any primer one of a), b) and c) including a radionuclide,
d) a solid material comprising a fluorescer which fluoresces when in proximity to the radionuclide
which method comprises using reagents a) and b) and optionally c) in the presence of a polymerase to form an amplified product based on the specific nucleic acid sequence, causing an aliquot of the said amplified product to bind to reagent d), and observing which method comprises using reagents a) and fluorescence emitted by reagent d) as a measure of the specific nucleic acid sequence. The specific nucleic acid sequence is also herein referred to as the target.

The present invention is illustrated with particular reference to nucleic acid amplification by PCR. This is a preferred amplification technique, however, the invention is applicable to other nucleic acid amplification techniques. Such techniques include for example those described in EP-A-0408295 and GB 2235689 which do not utilise a thermal cycler to denature the nucleic acid strands as is used in the PCR technique. Techniques known in the art which can be used in the method the invention include Nucleic Acid Sequence Based Amplification (NASBA), Strand Displacement Amplification (SDA) and Q-β Replicase Amplification.

The Ligase Chain Reaction (LCR) is a further amplification technique that may be employed. LCR involves the use of two primers for each strand of nucleic acid to be amplified, these being ligated in the presence of the specific sequence to produce a complementary strand.

Thus the invention also provides a method of amplifying and quantitating a specific nucleic acid sequence in a sample, by the use of
ab) a pair of oligonucleotide primers for each strand of the sequence to be amplified, each pair of primers together being complementary to the whole sequence strand, one primer of the pair required for one strand being labelled with a radiolabel and the other primer of the pair labelled with a capture label,
d) a solid material comprising a fluorescer which fluoresces when in proximity to the radionucleotide,
which method comprises using reagent ab) in the presence of a ligase to form an amplified product based on the specific nucleic acid sequence, causing an aliquot of the said amplified product to bind to reagent d), and observing fluorescence emitted by reagent d) as a measure of the specific nucleic acid sequence.

Reagent d) is a solid material comprising a fluorescer which fluoresces when in proximity to a radionuclide. The material may take various physical forms; a massive surface such as the surfaces of wells in a microtitre plate or other reaction vessel; or a particulate surface such as fibres or beads. Beads for this purpose are commercially available and are known as scintillation proximity assay (SPA) beads. Assay beads and methods suitable for use in the present invention are described in US Patent 4568649.

Impregnated or otherwise attached to the solid surface is a fluorescer which fluoresces in response to radioactive disintegrations. The SPA technique involves use of a radionuclide whose radiation, eg beta particles or Auger electrons, has such a short pathlength that only radionuclides immobilised on the surface cause fluorescence. The aqueous medium acts as a scavenger to absorb radiation from radionuclides in solution and so prevent it giving rise to fluorescence. Thus the SPA technique permits quantitation of radionuclide adsorbed on the solid surface, even in the presence of radionuclide in the surrounding fluid medium.

In a preferred method according to the invention, reagent d) carries on its surface a specific binder for the amplified product. One of reagents a), b) and c) which does not include a radionuclide, may comprise a capture label which binds to the specific binder of reagent d).

The capture label may be any detectable group for which a specific binder is available. Preferred examples are a biotin capture label which is bound by streptavidin and an antigen/antibody combination. However, any other combination of a capture label and binder may be used such as a hormone/receptor combination or an enzyme/substrate combination. Other suitable capture label/binders would be apparent to the skilled worker. The primer or probe would normally be capture labelled at the 5' end. It will also be apparent that the reagent a), b) or c) could be capture labelled with the binder and the group to which the binder binds would then be attached to reagent d), the solid material comprising fluorescer.

A number of different combinations of capture label/binder provisions are within scope of the invention. These include the following possibilities:
(i) at least one of the oligonucleotide primers a) comprises the capture label, at least one of the nucleotides b) includes a radionuclide and the optional probe c) is not used.
(ii) at least one of the oligonucleotide primers a) comprises the capture label and a probe c) is included and comprises a radionuclide.
(iii) at least one of the oligonucleotide primers a) comprises a radionuclide, at least one of the nucleotides b) comprise the capture label and the optional probe c) is not included.
(iv) at least one of the oligonucleotide primers a) comprises a radionuclide and a probe c) is included and comprises the capture label.
(v) at least one of the nucleotides b) includes a radionuclide and a probe c) is included and comprises the capture label.
(vi) at least one of the nucleotides b) comprises the capture label and a probe c) is included and comprises a radionuclide.

It should be noted that the method according to the invention can be carried out without using a capture label/binder interaction. This aspect of the invention results from the surprising discovery that accurate quantitation of a nucleic acid sequence can be achieved by the present technique relying on non-specific binding of nucleic acid amplification products to reagent d), the solid material. It can be seen that the radionuclide would be included in the nucleotide(s) b).

Non-specific binding of amplified products is believed to be due to the solid material d) having a positive charge and the phosphate backbone of DNA or RNA giving it an overall negative charge. Nucleic acids will therefore bind to reagent d) while separate nucleotides will not bind. Bound specific nucleic acid sequence or its amplified product containing radionuclide will cause the fluorescer in d) to fluoresce.

Use of a hybridization probe in a SPA -PCR assay avoids the possibility of generating false positive signals as a result of primer-dimer formation and also provides a built-in method for identification of the PCR product hence avoiding the need for gel electrophoresis as a means of demonstrating PCR specificity. When the preferred possibility iv) above is used, the capture labelled probe c) may be attached to the solid material d) before or after being hybridised to the amplified nucleic acid sequence. The same applies for possibility ii) above, where capture labelled primers may be attached to the solid material d) before or after the radiolabelled probe c) is hybridised to the amplified product.

An alternative method for demonstrating specificity of amplification is the use of a restriction enzyme cutting site within the amplified product. Cutting at this site will result in loss of signal proportional to the distance from the capture labelled end if the correct product has been made. In this example a specific capture system is required which must be present on only one of the two primers, the radionuclide being incorporated into the nucleotides.

A preferred radionuclide label is [³H] tritium although [¹²⁵I] iodine can also be used by virtue of its Auger electron emission. Even fairly energetic beta-emitters such as [¹⁴C] carbon, [³⁵S] sulphur and [³³P] phosphorus can be used. When the radionuclide is incorporated into one or more of the nucleotides in the supply of nucleotides b), the radiolabelled nucleotides are incorporated into the extension products at each cycle of amplification and thus multiple radiolabels are incorporated into each extension product.

Once the final cycle of amplification has been completed a portion of the product mixture is contacted with the solid material d), which contains a fluorescer and may be in the form of scintillation proximity assay beads.

Use of radionuclides which are high energy radiation emitting isotopes, such as [³²P]phosphorus for example, may be unsatisfactory, if the longer pathlength of the high energy β particles from ³²P results in very high background signals not only from the fluormicrosphere beads but also from Cerenkov radiation produced in solution.

Quantitation of an unknown sample can be performed by comparing to a standard curve constructed using known numbers of the target nucleic acid. In the PCR reaction, amplification reaches a limiting level once product reaches a plateau (14) and with higher amounts of starting target this "plateau" is attained after fewer cycles than with low amounts of starting target. As a consequence the accuracy of quantitation above this level is subsequently reduced. By reducing or increasing the number of PCR cycles run one can vary the overall amplification and hence the minimum number of target copies to obtain amplification to a "plateau" level can be adjusted according to the assay performed.

Careful consideration has also been given to optimising the concentrations of primer and nucleotides to avoid formation of non specific products, for example, "primer dimers" (amplification of the primers when annealed, fortuitously, to each other and not to the target).

In order to minimise the effects of primer-dimers, which would be detected as a false positive by SPA-PCR, it is sometimes necessary to reduce primer concentrations, from a standard PCR concentration of 1µM, to generally less than 0.5µM and usually 0.2µM or 0.1µM or less. The number of cycles for each target is carefully optimised to avoid amplifying all target copies to the "plateau" described earlier.
Correspondingly by varying the number of PCR cycles it is possible to change the working range of the assay to encompass all ranges of target amount depending upon the application.

In order to overcome concerns regarding sample consistency, for example using unextracted DNA samples, an internal control for each amplification can be included by co-amplifying a known, different, target using, for example, a fluorescein 5'-labelled primer and SPA beads coated with anti-fluorescein antibody.

By use of a thermostable DNA polymerase such as Taq and programmable heating blocks the PCR is readily amenable to automation. The method of the present invention is highly advantageous over previously reported PCR quantitation methods in that there is no separation and washing stage required. Once PCR is complete only one reagent is generally required, the assay beads, which may be added to the reaction product mixture or an aliquot of the product mixture added to the beads and the sample is then ready for counting. This step may be easily handled by automative sampling and pipetting apparatus currently available. Consequently results can be obtained with a very much shorter time than is possible with previous quantitation methods.

The Polymerase Chain Reaction is being widely adopted as a method of analysis (15). Once a rapid and reliable method for quantitation is available its usefulness in, for example, clinical diagnostics will be greatly increased. There is currently a requirement for quantitative PCR so that genetic and viral detection by PCR can be linked to an understanding of disease progress/remission (16,17).

The novel SPA-PCR method presented here affords a rapid and quantitative detection of PCR products that can be directly related to initial quantities of target DNA. In contrast to other methods of quantitative PCR which are multistep, time consuming and manually intensive, this method uses two steps and is rapid. It easily lends itself to automation for the large scale screening of clinical samples. Furthermore, when the nucleotides are radiolabelled the amount of radiolabel incorporated is proportional to the size of the labelled molecules and so background "noise" due to primer-dimer labelling is much less significant than for other methods which employ a single labelled moiety per molecule.

Other techniques such as the Ligase Chain reaction, like PCR, give exponential amplification of target sequence. In the case of LCR, the target is restricted to the sequence covered by pairs of oligonucleotides (1 pair per target strand of DNA) which hybridise adjacent to each other, generally covering about 60 base pairs total. A full review of the LCR is presented by F. Barany in "PCR, Methods and Applications", August 1991, Vol. 1, Number 1, Page 5. A specific application of LCR is presented by Barany in P.N.A.S., Vol. 88, pp, 189-193, January 1991.

Using SPA, it is possible to quantify the product generated during a LCR reaction and relate that to the original copy number of target sequence. This can be achieved as in the Example described later by labelling the 3'OH end of the one of the adjacent oligonucleotides (the Radiolabelled Oligonucleotide) with a radioactive moiety, such as ³H/³⁵S/³³P labelled nucleotides, and attaching a specific capture label such as biotin or antigen to the 5'PO₄ end of the other adjacent oligonucleotide (the Capture Oligonucleotide). In the event of ligation, the Radiolabelled Oligonucleotide will become covalently attached to the Capture Oligonucleotide, forming the ligation product which is specifically captured and counted on SPA beads. The ligation reaction occurs on both strands of target duplex DNA, hence the need for two pairs of oligonucleotides and the exponential nature of the LCR.

A further technique, NASBA, is a simple and rapid method for continuous amplification of nucleic acids in a single mixture at one temperature. Two primers are used, the first incorporating a promoter sequence recognized by an RNA polymerase and the second being derived from the opposite side of the target sequence to the first primer. The technique is described in PNAS (1990) 87, 1874-8.

PCR results in exponential accumulation of target sequence therefore it requires approximately 20 cycles to enable an amplification of one million fold. With NASBA however only 4-5 cycles would be required to produce equivalent amplification as 10-100 copies of RNA are generated at each transcription step. Double-stranded nucleic acids can be used with NASBA as well as single-stranded RNA but double-stranded templates must first be converted to single-stranded templates in order to be compatible with the process. Either primer (or both) can be biotinylated for capture and subsequent detection by SPA. Radiolabelled nucleotides can be used in the nucleic acid synthesis reaction.

SDA is an isothermal alternative for amplification of DNA using a restriction enzyme/DNA polymerase system. This technique is described in detail in PNAS (1992) 89, 392-396.

Both double-stranded and single-stranded target DNA fragments can be used with this technique and as with PCR high levels of target amplification can be achieved. In relation to SPA quantitation assays the standard method of using biotinylated primers cannot always be employed. A system would have to be used in which the label was incorporated into the DNA product. This could involve use of radiolabelled nucleotides and a capture hybridization method, or radiolabelled and capture labelled nucleotides, or radiolabelled nucleotides with total DNA capture.

Q-β Replicase amplification relies on the ability of the enzyme Q-β replicase to amplify exponentially recombinant RNA molecules. This technique exponentially amplifies probe sequences rather than target sequences as in the PCR.

Studies of Q-β replicase have shown that highly specific single-stranded RNA will serve as template for the synthesis of complementary single-stranded products. Following strand synthesis the template and product are released from the replication complex and both strands are then available for subsequent rounds of replication and therefore the number of RNA strands can increase exponentially. The technique is described in detail in Nucleic Acids Research (1986), 14 (14), 5591-5603. In SPA quantification of Q-β Replicase amplification the primer is not incorporated into the amplified product. The primer will not therefore include either the radionuclide or a capture label.

The invention is further illustrated in the following Examples.

### EXAMPLE 1

Quantitation of target copies of a marker region adjacent to the cystic fibrosis gene.
A 386 bp region was amplified using the primers (13):

1. 5´ Biotin AAT GCA ACA ATT CAC CCA ATT GCT CA 3´

2.   5´ GGT TAG GTC AGA GAA CAA AGC AAA TT 3´

Primers were obtained from Medprobe, Oslo, Norway. The biotinylated primer was HPLC purified by Medprobe. PCR was performed using the following optimised reagent concentrations 10mM Tris HCl, pH9.0, 50mM KCl, 1.5mM MgCl₂ 100-µg/ml gelatin, 20µM dATP, dCTP, dGTP, 20µM ³H TTP (lCi/mmol), 0.1µM primers and 2 units of Taq/50µl mix.

Reagents were from Sigma, Poole, UK, ³H TTP and Taq were from Amersham, UK. 2.5ml of the above was prepared ("master mix") to standardise the PCR mix. 5µl of a solution of human genomic DNA containing 0-20ng/µl was placed in a 500µl eppendorf tube and 45µl of master mix added. The solution was then overlaid with 2 drops of light mineral oil (Sigma) and thermal cycling performed using a Biometra Trio Thermoblock (Biometra, Maidstone, Kent, UK).

The initial denaturation was at 95°C for 30 seconds followed by 40 cycles of 55°C for 2 minutes then 72°C for 3 minutes then 95°C for 30 seconds. A final extension of 55°C for 2 minutes then 72°C for 10 minutes was performed after the last cycle.

After amplification 25µl of the reaction mix was removed and added to 25µl of an optimised streptavidin coated polyvinyltoluene SPA beads (0.25mg) in 500µl of 50mM Na₂ EDTA pH7. The mix was counted, without any further incubation, for 15 seconds on an LKB Rackbeta 1209 scintillation counter with a 0-999 window.

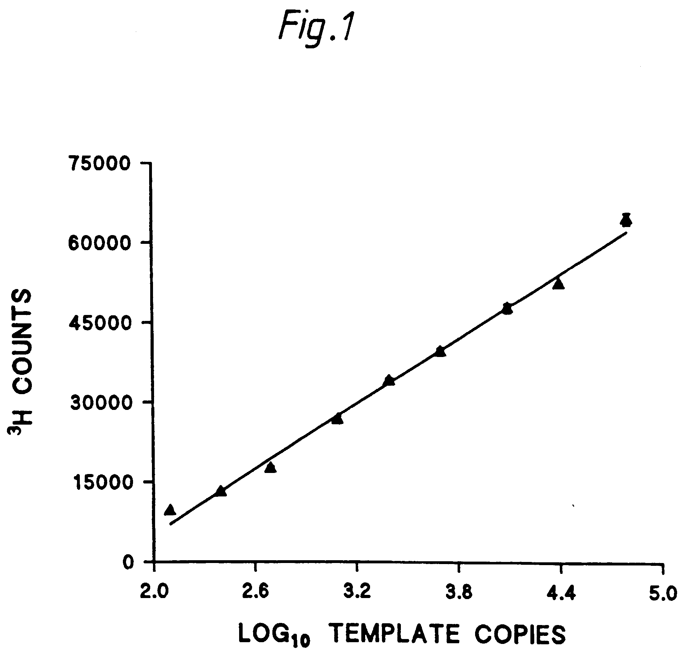

Results are shown in Figure 1.

### EXAMPLE 2

Quantitation of the human β-globin gene by amplification of a 268bp region of the gene.

A 268bp target of the β-globin gene was amplified using primers PCO4 and GH2O (Cetus Corp, Emeryville, California, USA) with the PCO4 primer biotinylated at the 5' end:

PCO4 5´ Biotin CAA CTT CAT CCA CGT TCA CC 3´

GH20 5´ GAA GAG CCA AGG ACA GGT AC 3´

Primers were synthesised using an Applied Biosystems PCR-Mate Oligonucleotide Synthesiser. 5' biotin was introduced using biotin phosphoramidite (Amersham).

Conditions for the PCR and analysis are as for Example 1 except that 35 cycles of amplification are performed with primers at 0.05µM concentration.

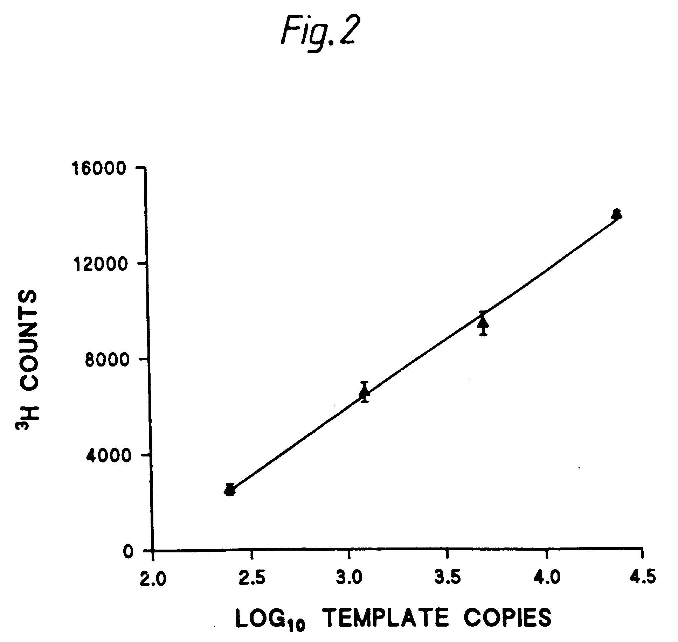

Results are shown in Figure 2.

Quantitation of target copies of β-globin is again achievable using the SPA-PCR. Accuracy is similar to that found with Example 1. The measured radioactivity in Example 2 is <50% of than in Example 1 and is due to:
i) the target sequence is shorter therefore less ³H TTP will be incorporated per product molecule, ii) the biotinylated primer has not been purified therefore some non biotinylated primer will be present to prime PCR but will not be bound subsequently to the SPA bead.

### EXAMPLE 3

Quantitation of p24 gene from plasmid pGEMEX containing the HIV1 p24 gene.

Serial dilutions of DNA from 17500 copies/5µl to 1.75 copies/5µl were prepared and 45µl of PCR mix added. PCR was performed as Example 1 except with primer concentration at 0.2µM and 40 cycles PCR with 2 units TAQ. The primers used were: 
5´ biotinylated SK39;TTT GGT CCT TGT CTT ATG TCC AGA ATG

and SK145;AGT GGG GGG ACA TCA AGC AGC CAT GCA AAT
 which amplify a 300bp region of the p24 gene of HIV-1

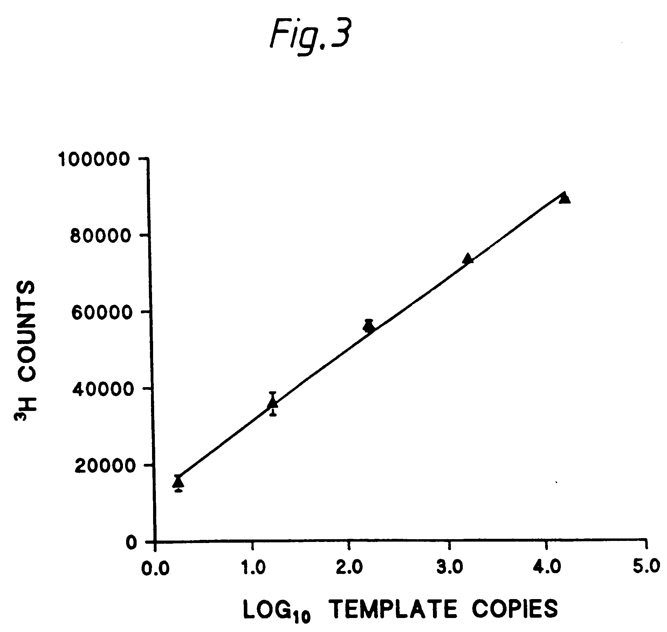

Results are shown in Figure 3.

Each point represents the mean +/- SEM of triplicate samples. The relationship between target copies and ³H counts is described by the line shown. The best fit line has an r value of 0.998.

### EXAMPLE 4

Quantification of copies of the plasmid PAT153 by amplification of a 500bp region of the plasmid and hybridization with a biotinylated hybridization probe.

The 500 bp target was amplified using the primers given below,

1. 5´GCG CTC ATC GTC ATC CTC GG 3´

2.    5´GAG GCC GTT GAG CAC CGC CG 3´

The primers were synthesized using an Applied Biosystems PCR-mate oligonucleotide synthesiser and purified using Oligonucleotide Purification Cartridges (Applied Biosystems) according to the manufacturer's protocol.

The PCR was performed as in Example 1.

Following amplification a 25µl aliquot of the PCR product was removed, denatured and hybridized to a third oligonucleotide probe which was biotinylated at its 5' end. The samples were heated at 95°C for 5 minutes and allowed to cool to room temperature in the presence of 1µM oligo (3). The sequence of this 25 mer probe occurs within the 500bp target region but it does not overlap the primer sequences. The sequence is given below,

3.    5´Biotin TAG AGG ATC CAC AGG ACG GGT GTG G 3´

The Probe was synthesized and purified as for primers (1) and (2). Biotin phosphoramidite (Amersham) was used to introduce the biotin group onto the 5' terminus.

After hybridization, 500µl of streptavidin coated polyvinyltoluene SPA beads in 100 mM MgC12 (final concentration of 2 mg/ml) was added and the samples counted for 20 seconds on a Rackbeta 1209 scintillation counter with a 0-999 window.

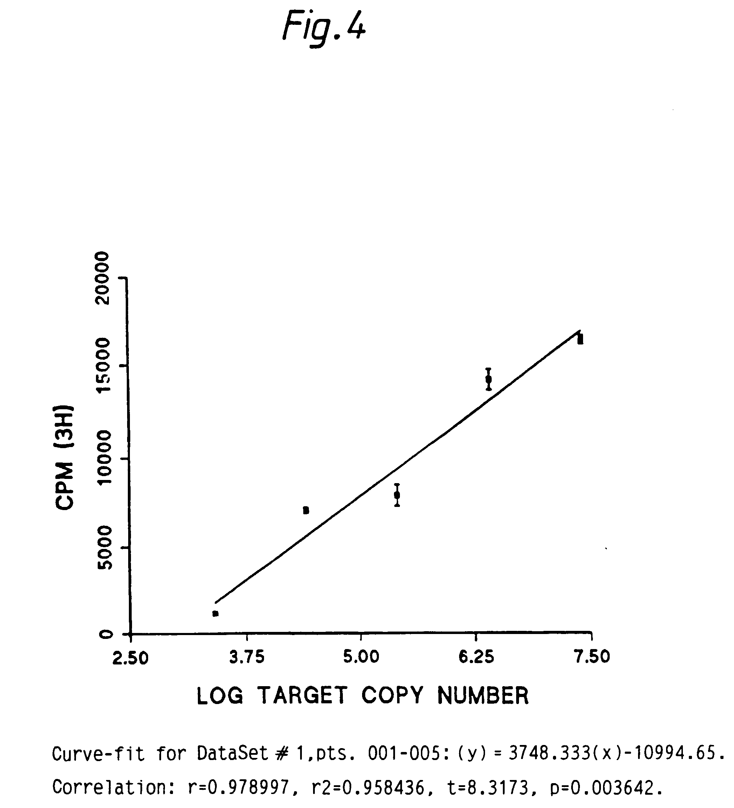

The results are shown in Figure 4.

Quantification was achieved over a wide range of copies i.e. 2.6x10³ up to 2.6x10⁷ copies. The counts obtained using the third oligo approach are lower than in the previous two examples. This is probably due to the fact that only half the PCR product is counted using this method. This could be rectified with the use of a second hybridization probe, complementary to the second strand of the PCR product but not to the first probe.

An alternative to the hybridization method outlined above would be the use of biotinylated primers to generate PCR product and following amplification, hybridization of radiolabelled oligonucleotide probe to an aliquot of denatured PCR product.

### EXAMPLE 5

Quantitation of target copies of a specific RNA. Synthetic GeneAmplimer pAW109 RNA (Perkin Elmer N808-0037) was amplified using the following pair of primers:-

### DM151 (Upstream)

5´ -BIOTIN GTC TCT GAA TCA GAA ATC CTT CTA TC - 3´

### DM152 (Downstream)

5′-BIOTIN CAT GTC AAA TTT CAC TGC TTC ATC C - 3′

This primer pair produces a 308 base pair product following RNA-PCR amplification of pAW109 RNA.

Primers were synthesized in-house using an ABI PCR-Mate oligonucleotide synthesizer. 5' biotin was introduced using biotin phosphoramidite (Amersham).

Primers were purified on ABI OPC cartridges according to manufacturer's protocol. Purified oligonucleotides were resuspended in sterile, distilled water at a final concentration of 50 µM. Storage was at -20°C.

RNA- PCR was performed using the following reagents which were added to 2 µl aliquots of pAW109 template - 10mM Tris HCl, pH 9.0, 50mM KCl, 1.5mM MgCl₂, 20µM dATP, dCTP, dGTP, 20µM ³H TTP (1 Ci/mmol), 0.1µM primer DM152, 10 units Reverse Transcriptase (Amersham RAV-2 Batch 3001). Final volume was adjusted to 20µl with pyrogen-free water and the mixture was incubated at 42°C for 30 minutes, then placed on ice. The volume was then adjusted to 100µl by the addition of the following reagents - 10mM Tris HCl, pH 9.0, 50mM KCl, 1.5mM MgCl₂, 100µg/ml gelatin, 20µM dATP, dCTP, dGTP, 20µM ³H TTP (1 Ci/mmol), 0.1µM primer DM151 and 2 units of Taq polymerase (Amersham), the mixture was adjusted to the 100µl final volume via the addition of sterile, distilled water. The complete reagent set required for the PCR was now present. All reagents were from Sigma, Poole, UK and ³H TTP was supplied by Amersham (TRK 576).

The reaction mix was overlaid with two drops of light mineral oil, followed by PCR over 50 cycles using identical amplification parameters to those in Example 1.

After amplification, 50µl of the reaction mix was removed and added to 500µl of 0.5 mg/ml streptavidin coated, polyvinyltoluene SPA beads in 50mM Na₂EDTA, pH 7.0. This assay mix was counted without further incubation for 20 seconds on an LKB Rackbeta 1209 scintillation counter with a 0-999 window. Also the remaining 50µl from each - 19 - sample was removed and analysed on a 1% agarose gel stained with 0.5µg/ml ethidium bromide.

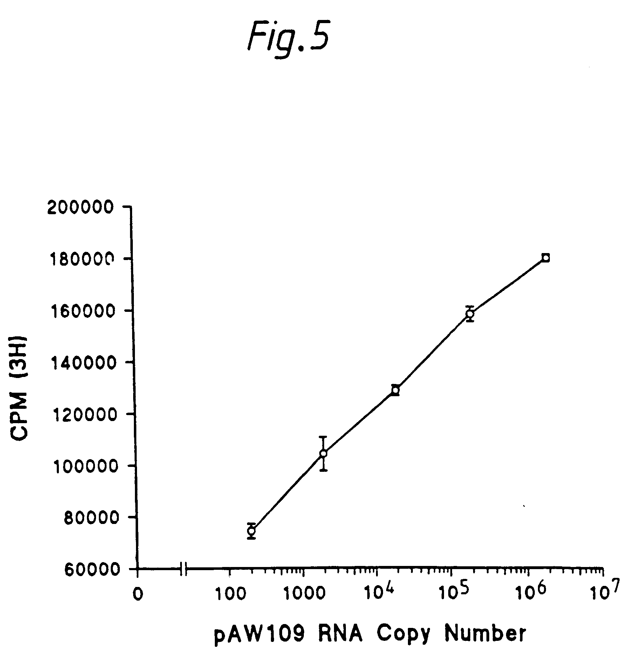

Results are shown in Figure 5.

Quantitation of RNA target copy numbers was therefore achievable using SPA-PCR.

### EXAMPLE 6

Quantitation of target copies of a marker region adjacent to the cystic fibrosis gene using an antigen - antibody method for capture of PCR product.

A 386bp region was amplified using the primers given in Example 1. The primers were not biotinylated and were synthesized using an Applied Biosystems PCR-mate oligonucleotide synthesizer and purified using Oligonucleotide Purification Cartridges (Applied Biosystems) according to the manufacturer's protocol. The PCR was performed as in Example 1 except the following mix was used:-20 µM dATP, dGTP, dCTP; 10µM ³H TTP (Amersham, UK; 1Ci/mmole) and 10 µM fluorescein dUTP.

Following amplification 30µl aliquots of PCR product were removed from each tube. The samples were incubated for 2 hours at room temperature with 170µl of 1x Phosphate Buffered Saline (Flow Laboratories, Irvine, Scotland) and 100µl of sheep anti-fluorescein antibody (Amersham) diluted 1:2500 in
PBS.Scintillation proximity assay anti-sheep reagent (Amersham) was reconstituted using 50 ml PBS and sonicated Herring sperm DNA was added to give a final concentration of 50µg DNA/100µl of bead suspension for the purpose of reducing background. 100µl of bead solution was added to each sample. The samples were mixed on an orbital shaker for 3 hours at room temperature and then counted for 20 seconds each on an LKB 1209 scintillation counter with the window open from 0-999.

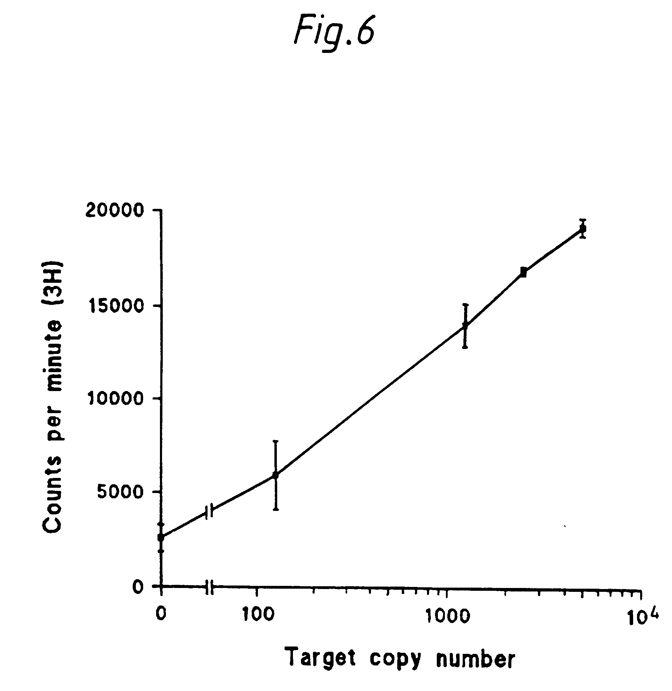

Results are shown in Figure 6.

Quantitation of target copies is therefore achievable using an SPA-PCR assay in which the capture label is an antigen and the binder is an antibody to that antigen. In this example quantitation was achieved in the range of 125 - 5000 copies.

### EXAMPLE 7

Quantitation of the wild-type Beta-globin gene (sickle-cell allele) using LCR.

The region was amplified using the following set of oligonucleotides:-

1.    5´ - BIOTIN GTC ATG GTG CAC CTG ACT CCT GA - 3´

2.        5´ - BIOTIN C TGC AGT AAC GGC AGA CTT CTC CT - 3´

3.    5´ G GAG AAG TCT GCC GTT ACT GCC - 3´

4.    5´ - C AGG AGT CAG GTG CAC CAT GGT - 3´

Where oligonucleotide number:-
1. Is the wild-type, anti-sense strand Capture Oligonucleotide.
2. Is the wild-type, sense strand Capture Oligonucleotide.
3. Is the wild-type, anti-sense strand Radiolabelled Oligonucleotide.
4. Is the wild-type, sense strand Radiolabelled Oligonucleotide.

All oligonucleotides were synthesized in-house using an ABI PCR-Mate oligonucleotide synthesizer. Oligonucleotides were purified using reverse-phase HPLC, resuspended at 50µM and stored at -20°C.

A 1mCi pack of ³H TTP (TRK 576, Amersham) was dried down under nitrogen, and resuspended in 100µl of sterile distilled water. This was used in the reaction mix outlined below.

Post-purification labelling reactions for the two Radiolabelled Oligonucleotides (3 and 4) were as follows - 25µl of each oligonucleotide (corresponding to 1250 pmoles of 3'OH ends) were added to 50µl of ³H TTP solution (-5000 pmoles, -0.5mCi ³H), 1.25µl of 5mM ddTTP (-1250 pmoles, Pharmacia), 20µl of five-times terminal transferase buffer (Amersham) and 5µl (10 units) of terminal transferase enzyme (Amersham). The final volume was adjusted to 100µl using sterile distilled water. The reaction mixes were incubated at 37°C for 2 hours. Incorporation efficiency was approximately 25% and the specific activity was 30 Ci/mmol of Radiolabelled Oligonucleotide. The final concentration of the Radiolabelled Oligonucleotides was 12.5µM.

The following reagent conditions were used per LCR assay - 0.1µM oligonucleotides 1/2/3/4, 20mM Tris HCL, pH7.6, 25mM potassium acetate, 10mM magnesium acetate, 10mM dithiothreitol, 0.6mM NAD and 0.1% Triton™ X-100, 10 units of Taq ligase enzyme (Ampligase, Cambio) and 5µl of diluted blood DNA from a normal, non-sickle-cell individual. Final volume was 50µl. The reaction mixes were overlaid with 2 drops of light mineral oil and the tubes loaded onto a Biometra Trio thermal-cycler. Cycling parameters were as follows - 95°C for 1 minute, 65°C for 4 minutes. This was repeated for 20 or 35 cycles.

Following cycling, 25µl aliquots of each sample were removed and added to 500µl of 4mg/ml polyvinyltoluene streptavidin-coated SPA beads in the presence of 50mM Na₂EDTA and 200mM NaOH. This mix was counted without further incubation for 20 seconds on an LKB Rackbeta scintillation counter with a 0-999 window.

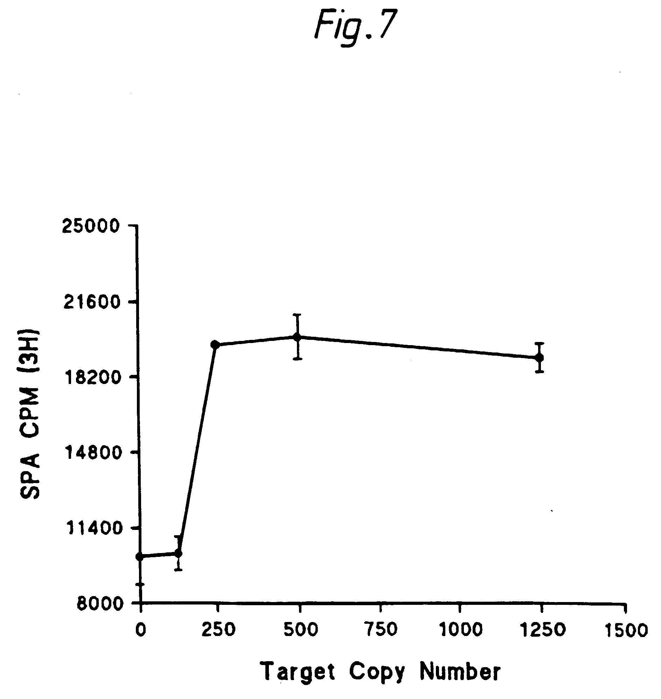

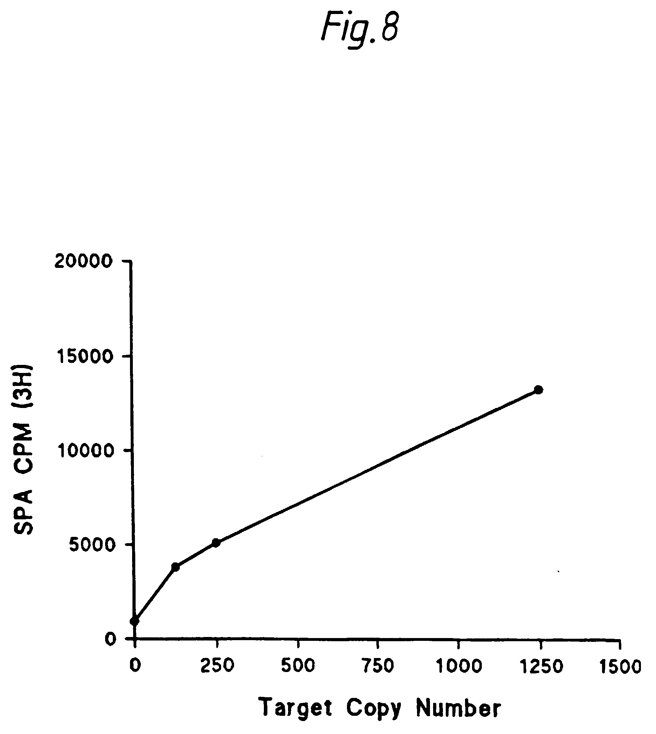

Results are shown in Figures 7 and 8. LCR over the range of DNA dilutions assayed has occurred and the SPA counts are reproducible and quantitative. In this test system, 35 cycles causes a distinct "plateau" effect (Figure 7), lower cycle numbers allows for an almost linear signal increase in response to initial copy number of target (Figure 8). This is analogous to the "plateau" observed during PCR reactions, above which accuracy of quantitation is reduced.

### EXAMPLE 8

Quantitation of target copies of a marker region adjacent to the cystic fibrosis gene using DNA binding properties exhibited by SPA beads.

The PCR was performed as in Example 1 except the primers were not biotinylated.

Following amplification, 30µl aliquots of PCR product were removed from each tube. 500µl of scintillation proximity assay reagent (yttrium silicate fluomicrospheres (Amersham, UK) in 1 x PBS) at a concentration of 1 mg yttrium silicate fluomicrospheres/500µl of buffer was added to each sample. The samples were incubated for 3 hours at room temperature on an orbital shaker and then counted for 20 seconds each on an LKB 1209 scintillation counter with the window open from 0-999.

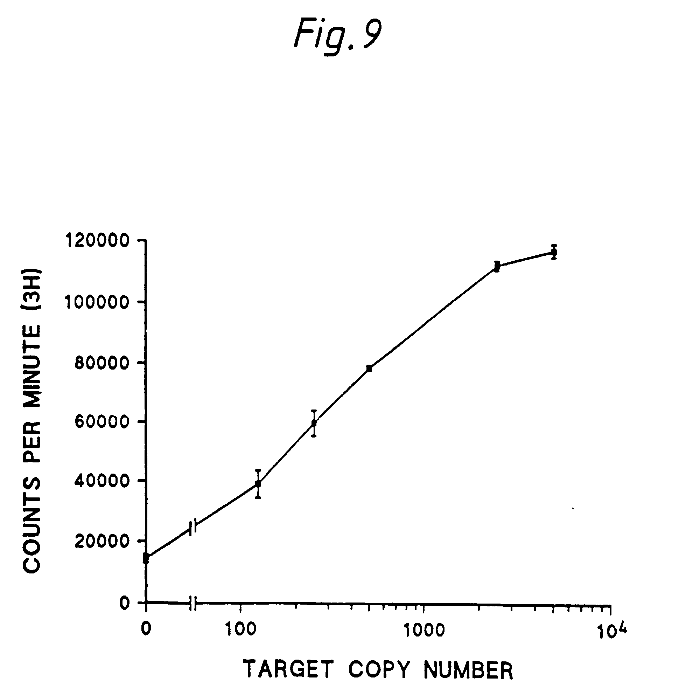

Results are shown in Figure 9.
*Fig. 1*
   CF SPA PCR, 0.1 µM PRIMERS, 2units TAQ
*Fig. 2*
   GLOBIN SPA PCR;35 CYCLES,0.05µM PRIMER,2U TAQ
*Fig. 3*
   HIV p24 GENE SPA PCR, 0.2µM PRIMERS, 2UNITS TAQ
*Fig. 4*
   HYBRIDIZATION CAPTURE OF PCR PRODUCT USING A BIOTINYLATED PROBE AND STREPTAVIDIN SPA BEADS. -20 µM Nucleotides, 0.1 µM primers, 2u Tag polymerase, 30 cycles, triplicate points for 1 experiment.
*Fig. 5*
   SPA RNA - PCR ASSAY USING pAW109 TEMPLATE
*Fig.6*
   QUANTITATION OF TARGET COPIES OF A MARKER REGION ADJACENT TO THE CYSTIC FIBROSIS GENE USING AN ANTIGEN-ANTIBODY SYSTEM FOR CAPTURE OF PCR PRODUCT.
*Fig. 7*
   SPA-LCR USING OLIGOS 1/2/3/4 AT 0.1µM 10 UNITS AMPLIGASE. 35 CYCLES BIOMETRA 4 mg/ml SA-SPA BEAD 50mM EDTA, 200mM NaOH. DUPLICATE POINTS.
*Fig. 8*
   SPA-LCR USING OLIGOS 1/2/3/4 AT 0.1µM 10 UNITS AMPLIGASE. 20 CYCLES BIOMETRA 4mg/ml SA-SPA BEAD 50mM EDTA, 200mN NaOH. MEANS OF DUPLICATES
*Fig. 9*
   QUANTITATION OF TARGET COPIES OF A MARKER REGION ADJACENT TO THE CYSTIC FIBROSIS GENE USING DNA BINDING PROPERTIES EXHIBITED BY SPA BEADS TO CAPTURE THE PCR PRODUCT.

### References:

1. Saiki. R., Scharf S., Faloona, F., Mullis, K., Horn, C., Erlich, H., Arnheim, *N. Science* (1985), 230, 1350.1354.
2. Cenesca, J. Yuan-Hu Yang, R., Alter H.J. and Wai-Kuo Shih, J., J. *Infectious Disease* (1990), 162, 1025-1030.
3. Dickover, R., Donovan, R., Goldstein, E., Dandekar, S., Bush, C., and Carlson, J., *J. Clin. Microbiology* (1990), 28, 9, 2130-2133.
4. Noonan, K., *et al, Proc. Natl. Acad. Sci* (1990), 87*,* 7160-7164.
5. Gilliland, C., Perrin, S., Blanchard, K., and Bunn, H. *Proc. Natl. Acad. Sci. (1990),* 87, 2725-2729.
6. Katz, J, Bodin, E., and Coen, D. *J. Virology* (1990), 64, 9, 4288-4295.
*7.* Bej. A., Steffan, R., Di Cesare, J., Haff, L., and Atlas, R. *Appl.* Env, *Microbiology* (1990), 56, 2, 307-314.
8. Kellogg, D.E., Sninsky, J., Kwok, S. *Anal. Biochemistry* (1990), 189, 202-208.
9. Rimstad, E., Hornes, E., Olsvik, O. and Hyllseth, B. *J. Clin. Microbiology* (1990), 28, 10, 2275-2278.
10. Lundeberg, J., Wahlberg, J., and Uhlen, M. *Biotechniques* (1991), 10, 1, 68-75.
11. Mullis, K., Faloona, F., Scharf, S., Saiki, K., Horn, C. and Erlich, H. *Cold Spring Harbor Symp. Quant. Biol.* (1986), 51, 263-273.
12. Bosworth, N. and Towers, P. *Nature* (1989), 341, 167-168.
13. Huth, A., Estivill, X., Crade, K., Billwitz, H., Speer, A., Rosenthal, A., Williamson, R., Ramsay, M, and Coutelle, C. *Nucleic Acid Res.* (1989), 17, 17, 7118.
14. Katz, E., and Haff, L. *Amplifications* (1989), 3, 8-9.
15. Wright, P, and Wynford Thomas, D. *J. pathology* (1990), 162, 99-117.
16. Eisenstein, B. *New Engl. J. Medicine* (1990), 322, 3,178-183,
17. Bréchot, C. *J. Hepatology* (1990), 11, 124-129.

## Claims

1. A method of amplifying and quantitating a specific nucleic acid sequence in a sample, by the use of
a) at least one oligonucleotide primer for each strand of the sequence to be amplified, the primers being complementary to part of the sequence strand but not to one another
b) a supply of nucleotides
c) optionally a probe complementary to part of the sequence to be amplified but not to any primer one of a), b) and c) including a radionuclide,
d) a solid material comprising a fluorescer which fluoresces when in proximity to the radionuclide
which method comprises using reagents a) and b) and optionally c) in the presence of a polymerase to form an amplified product based on the specific nucleic acid sequence, causing an aliquot of the said amplified product to bind to reagent d), and observing fluorescence emitted by reagent d) as a measure of the specific nucleic acid sequence.

2. A method as claimed in claim 1, wherein the specific nucleic acid sequence is amplified by the Polymerase Chain Reaction technique.

3. A method as claimed in claim 1 or claim 2, wherein reagent d) carries on its surface a specific binder for the amplified product.

4. A method as claimed in claim 3, wherein one of reagents a), b) and c) which does not include a radionuclide, comprises a capture label which binds to the specific binder of reagent d).

5. A method as claimed in claim 4, wherein at least one of the oligonucleotide primers a) comprises the capture label, at least one of the nucleotides b) includes a radionuclide, and the optional probe c) is not used.

6. A method as claimed in claim 4, wherein the probe c) comprises the capture label.

7. A method as claimed in any one of claims 4 to 6, wherein the capture label is biotin and the binder is streptavidin.

8. A method according to any one of claims 4 to 6, wherein the capture label is an antigen and the binder is an antibody to that antigen.

9. A method according to claim 1 or claim 2 wherein the amplified product binds to reagent d) in a non-specific way.

10. A method according to any one of claims 1 to 8, wherein the radionuclide is chosen from [³H] tritium, [¹²⁵I] iodine, [¹⁴C] carbon, [³⁵S] sulphur and [³³P] phosphorus.

11. A method of amplifying and quantitating a specific nucleic acid sequence in a sample, by the use of
ab) a pair of oligonucleotide primers for each strand of the sequence to be amplified, each pair of primers together being complementary to the whole sequence strand to be amplified, one primer of the pair required for one strand being labelled with a radiolabel and the other primer of the pair labelled with a capture label,
d) a solid material comprising a fluorescer which fluoresces when in proximity to the radionucleotide,
which method comprises using reagent ab) in the presence of a ligase to form an amplified product based on the specific nucleic acid sequence, causing an aliquot of the said amplified product to bind to reagent d), and observing fluorescence emitted by reagent d) as a measure of the specific nucleic acid sequence.

12. A method according to any one of claims 1 to 11, wherein the number of copies of the specific nucleic acid sequence initially present is estimated by reference to a standard curve constructed using samples containing known copy numbers of the specific sequence.

13. A method according to claim 12, wherein the extent of amplification is adjusted such that the amplified product concentration falls within a region of the standard curve below a plateau region.

14. A method as claimed in any one of claims 1 to 13, wherein the solid material d) is in the form of scintillation proximity assay beads.

## Patentansprüche

1. Verfahren zur Amplifikation und quantitativen Bestimmung einer spezifischen Nucleinsäuresequenz in einer Probe, unter Verwendung von
a) zumindest einem Oligonucleotidprimer für jeden Strang der zu amplifizierenden Sequenz, wobei die Primer zu einem Teil des Sequenzstrangs aber nicht zueinander komplementär sind
b) einem Vorrat an Nucleotiden
c) gegebenenfalls einer Sonde, die komplementär ist zu einem Teil der zu amplifizierenden Sequenz, aber nicht zu einem Primer,
wobei entweder a), b) oder c) ein Radionuklid umfaßt,
d) einem Feststoff, umfassend eine fluoreszierende Verbindung, die fluoresziert, wenn sie sich in der Nähe des Radionuklids befindet,
wobei das Verfahren umfaßt, daß die Reagenzien a) und b) und gegebenenfalls c) in Gegenwart einer Polymerase zur Bildung eines amplifizierten Produkts verwendet werden, das auf der spezifischen Nucleinsäuresequenz basiert, ein Aliquot des amplifizierten Produkts mit dem Reagenz d) zur Bindung gebracht wird, und die von dem Reagenz d) emittierte Fluoreszenz als Maß für die spezifische Nucleinsäuresequenz beobachtet wird.

2. Verfahren nach Anspruch 1, wobei die spezifische Nucleinsäuresequenz durch die Polymerase-Ketten-Reaktionstechnik amplifiziert wird.

3. Verfahren nach Anspruch 1 oder 2, wobei das Reagenz d) auf seiner Oberfläche einen spezifischen Binder für das amplifizierte Produkt trägt.

4. Verfahren nach Anspruch 3, wobei eines der Reagenzien a), b) und c), das kein Radionuklid enthält, eine Einfang-Markierung umfaßt, die an den spezifischen Binder des Reagenz d) bindet.

5. Verfahren nach Anspruch 4, wobei zumindest einer der Oligonucleotidprimer a) die Einfang-Markierung umfaßt, zumindest eines der Nucleotide b) ein Radionuklid umfaßt, und die fakultative Sonde c) nicht verwendet wird.

6. Verfahren nach Anspruch 4, wobei die Sonde c) die Einfang-Markierung umfaßt.

7. Verfahren nach einem der Ansprüche 4 bis 6, wobei die Einfang-Markierung Biotin ist und der Binder Streptavidin ist.

8. Verfahren nach einem der Ansprüche 4 bis 6, wobei die Einfang-Markierung ein Antigen ist und der Binder ein Antikörper zu diesem Antigen ist.

9. Verfahren nach Anspruch 1 oder 2, wobei das amplifizierte Produkt auf nicht-spezifische Weise an das Reagenz d) bindet.

10. Verfahren nach einem der Ansprüche 1 bis 8, wobei das Radionuklid ausgewählt wird aus [³H] Tritium, [¹²⁵I] Iod, [¹⁴C] Kohlenstoff, [³⁵S] Schwefel und [³³P] Phosphor.

11. Verfahren zur Amplifikation und quantitativen Bestimmung einer spezifischen Nucleinsäuresequenz in einer Probe, unter Verwendung von
ab) einem Paar Oligonucleotidprimer für jeden Strang der zu amplifizierenden Sequenz, wobei jedes Primerpaar zusammen komplementär zu dem gesamten zu amplifizierenden Sequenzstrang ist, ein Primer des für einen Strang benötigten Primerpaares mit einer Radio-Markierung markiert ist und der andere Primer des Primerpaares mit einer Einfang-Markierung markiert ist,
d) einem Feststoff, umfassend eine fluoreszierende Verbindung, die fluoresziert, wenn sie sich in der Nähe des Radionucleotids befindet,
wobei das Verfahren umfaßt, daß das Reagenz ab) in Gegenwart einer Ligase zur Bildung eines amplifizierten Produkts verwendet wird, das auf der spezifischen Nucleinsäuresequenz basiert, ein Aliquot des amplifizierten Produkts mit dem Reagenz d) zur Bindung gebracht wird, und die von dem Reagenz d) emittierte Fluoreszenz als Maß für die spezifische Nucleinsäuresequenz beobachtet wird.

12. Verfahren nach einem der Ansprüche 1 bis 11, wobei die Anzahl der ursprünglich vorhandenen Kopien der spezifischen Nucleinsäuresequenz durch Bezug zu einer Standardkurve geschätzt wird, die unter Verwendung von Proben mit einer bekannten Kopienanzahl der spezifischen Sequenz erstellt wurde.

13. Verfahren nach Anspruch 12, wobei der Amplifikationsgrad so eingestellt wird, daß die Konzentration des amplifizierten Produkts innerhalb eines Bereichs der Standardkurve unterhalb eines Plateaubereichs liegt.

14. Verfahren nach einem der Ansprüche 1 bis 13, wobei der Feststoff d) in Form von "Scintillation proximity assay beads" vorliegt.

## Revendications

1. Procédé d'amplification et de quantification d'une séquence d'acide nucléique spécifique dans un échantillon, par l'utilisation :
a) d'au moins une amorce oligonucléotidique pour chaque brin de la séquence devant être amplifiée, les amorces étant complémentaires de la partie du brin de la séquence mais non complémentaire l'une de l'autre
b) d'un apport de nucléotides
c) de manière optionnelle d'une sonde complémentaire de la partie de la séquence devant être amplifiée mais complémentaire d'aucune amorce
de a), b) et c) comportant un radioélément,
d) d'un matériel solide comportant un agent fluorescent qui est fluorescent lorsqu'il est situé à proximité du radioélément,
lequel procédé comporte l'utilisation des réactifs a) et b) et de manière optionnelle c) en présence d'une polymérase pour former un produit amplifié basé sur la séquence d'acide nucléique spécifique, la formation d'une liaison d'une partie aliquote dudit produit amplifié au réactif d), et l'observation de la fluorescence émise par le réactif d) en tant qu'une mesure de la séquence d'acide nucléique spécifique.

2. Procédé selon la revendication 1, dans lequel la séquence d'acide nucléique spécifique est amplifiée par la technique de Réaction de Polymérisation en Chaîne.

3. Procédé selon la revendication 1 ou 2, dans lequel le réactif d) porte à sa surface un agent de liaison spécifique au produit amplifié.

4. Procédé selon la revendication 3, dans lequel un des réactifs a), b) et c) qui n'inclut pas de radioélément, comporte un marqueur de capture qui se lie à l'agent de liaison spécifique du réactif d).

5. Procédé selon la revendication 4, dans lequel au moins une des amorces oligonucléotidiques a) comporte le marqueur de capture, au moins un des nucléotides b) comporte un radioélément, et la sonde optionnelle c) n'est pas utilisée.

6. Procédé selon la revendication 4, dans lequel la sonde c) comporte le marqueur de capture.

7. Procédé selon les revendications 4 à 6, dans lequel le marqueur de capture est la biotine et l'agent de liaison est la streptavidine.

8. Procédé selon l'une quelconque des revendications 4 à 6, dans lequel le marqueur de capture est un antigène et l'agent de liaison est un anticorps dirigé contre cet antigène.

9. Procédé selon la revendication 1 ou 2, dans lequel le produit amplifié se lie au réactif d) d'une manière non-spécifique.

10. Procédé selon l'une quelconque des revendications 1 à 8, dans lequel le radioélément est choisi parmi le groupe constitué de tritium [H³], d'iode [I¹²⁵], de carbone [C¹⁴], de soufre [S³⁵] et de phosphore [P³²].

11. Procédé d'amplification et de quantification d'une séquence d'acide nucléique spécifique dans un échantillon, par l'utilisation :
ab) d'une paire d'amorces oligonucléotidiques pour chaque brin de la séquence devant être amplifiée, les deux amorces de chaque paire étant ensemble complémentaires de la totalité du brin de la séquence devant être amplifiée, une des amorces de la paire nécessaire à un brin étant marquée à l'aide d'un marqueur isotopique et l'autre amorce de la paire étant marquée à l'aide d'un marqueur de capture,
d) d'un matériel solide comportant un agent fluorescent qui est fluorescent lorsqu'il est situé à proximité du radioélément,
lequel procédé comporte l'utilisation du réactif ab) en présence d'une ligase pour former un produit amplifié basé sur la séquence d'acide nucléique spécifique, la formation d'une liaison d'une partie aliquote dudit produit amplifié au réactif d), et l'observation de la fluorescence émise par le réactif d) en tant qu'une mesure de la séquence d'acide nucléique spécifique.

12. Procédé selon l'une quelconque des revendications 1 à 11, dans lequel le nombre de copies de la séquence d'acide nucléique spécifique initialement présente est estimée par rapport à une courbe standard construite en utilisant des échantillons contenant des nombres de copies connus de la séquence spécifique.

13. Procédé selon la revendication 12, dans lequel l'extension de l'amplification est ajustée de sorte que la concentration du produit amplifié soit comprise dans une région de la courbe standard située en dessous d'une région plateau.

14. Procédé selon l'une quelconque des revendications 1 à 13, dans lequel le matériel solide d) se présente sous la forme de billes d'essai de proximité de scintillation.
